# EUROPEAN PATENT SPECIFICATION

(11) **EP 2 780 027 B1**
(45) Date of publication and mention of the grant of the patent: **19.07.2017**
(21) Application number: 12795955.9
(22) Date of filing: 15.11.2012
(51) Int. Cl.: A61K 38/17, A61P 25/28

(54) **APOAEQUORIN FOR REDUCING NEURONAL INJURY DUE TO ISCHEMIA**
APOAEQUORIN ZUR VERRINGERUNG EINES ISCHÄMIEBEDINGTEN NEURONALEN SCHADENS
APOAÉQUORINE POUR RÉDUIRE UNE LÉSION NEURONALE DUE À UNE ISCHÉMIE

(30) Priority: 15.11.2011 US 201161559816 P
(43) Date of publication of application: 24.09.2014
(73) Proprietor: Quincy Bioscience LLC, Madison, WI 53717 (US)
(72) Inventor: UNDERWOOD, Mark, Y., Madison, WI 53717 (US); MOYER, James, R., Milwaukee, WI 53201 (US)
(74) Representative: advotec.
(86) International application number: PCT/US2012/065291
(87) International publication number: WO 2013/074798

(56) References cited:
- WO-A1-2009/114597
- US-A1- 2011 130 336
- HOCHSTETTER E L ET AL: "Apoaequorin protects neurons from ischemia and alters cytokine mRNA levels in rat hippocampus.", SOCIETY FOR NEUROSCIENCE ABSTRACT VIEWER AND ITINERARY PLANNER, vol. 42, 2012, XP009166941, & 42ND ANNUAL MEETING OF THE SOCIETY-FOR-NEUROSCIENCE; NEW ORLEANS, LA, USA; OCTOBER 13 -17, 2012
- DETERT J A ET AL: "Neuroprotection of hippocampal CA1 neurons from ischemic cell death using the calcium binding protein aequorin", SOCIETY FOR NEUROSCIENCE ABSTRACT VIEWER AND ITINERARY PLANNER, vol. 39, 2009, XP009166942, & 39TH ANNUAL MEETING OF THE SOCIETY-FOR-NEUROSCIENCE; CHICAGO, IL, USA; OCTOBER 17 -21, 2009 cited in the application
- DETERT J A ET AL: "Time course and effectiveness of apoaequorin as a neuroprotectant in the brain.", SOCIETY FOR NEUROSCIENCE ABSTRACT VIEWER AND ITINERARY PLANNER, vol. 41, 2011, XP009166943, & 41ST ANNUAL MEETING OF THE SOCIETY-FOR-NEUROSCIENCE; WASHINGTON, DC, USA; NOVEMBER 12 -16, 2011

## Description

This application claims the benefit of U.S. Application No. 61/559,816, filed November 15,2011.

### FIELD OF THE INVENTION

This disclosure relates to compositions for reducing neuronal injury due to ischemia. In particular, this disclosure is directed to apoaequorin and its use in reducing neuronal injury due to brain ischemia in a subject.

### BACKGROUND OF INVENTION

According to the American Heart Association, every 40 seconds, someone in the United States suffers from a stroke. Currently, there is only one Food and Drug Administration approved treatment for stroke, recombinant tissue plasminogen activator (rTPA). Although rTPA helps many people, it can also have deleterious side effects, such as hemorrhage. During ischemia, neurons are subjected to an excess of intracellular calcium. Although intracellular calcium is necessary for normal neuronal functions, too much calcium can trigger cascades of events, leading up to, and including cell death. Several mechanisms enable neurons to limit or control cytosolic calcium levels, including calcium binding proteins (CaBPs). It has been shown that treatment with the CaBP calbindin D-28k reduces deleterious effects from ischemia. Unfortunately, there is a lack of alternative therapeutics for preventing neuron death due to ischemia and, accordingly, there exists a need for novel therapeutics useful in treating the deleterious effects of ischemia on neurons. The invention is defined in the claims. Here, the inventors demonstrate the use of apoaequorin for providing ischemic protection to neurons. Accordingly, the disclosure encompasses in a first aspect apoaequorin for use in preconditioning neurons in a subject, preferably human, to reduce neuronal injury due to brain ischemia. Such a use includes administering apoaequorin to the subject, wherein the apoaequorin initiates a change in cytokine expression levels in the neurons that results in a reduction in neuronal injury due to brain ischemia.

The administration is performed by injection into area CA1 of dorsal hippocampus 48 hours before the brain ischemia occurs.

In certain embodiments, the reduction in neuronal injury effect lasts at least 24 hours from the time of apoaequorin administration. In other embodiments, the reduction in neuronal injury effect lasts at least 48 hours from the time of apoaequorin administration.

Further disclosed is the use of apoaequorin for the manufacture of a medicament for preconditioning neurons in a subject to reduce neuronal injury due to brain ischemia.

The invention further encompasses a composition for use in preconditioning neurons in a subject to reduce neuronal injury due to brain ischemia with the features of claim 7. Preferred compositions are formulated as injectable dosages.

Further disclosed is a kit for preconditioning neurons in a subject to reduce neuronal injury due to brain ischemia. Such a kit includes: (a) a therapeutically effective dosage of apoaequorin to precondition neurons in a subject to reduce neuronal injury due to brain ischemia; and (b) a delivery device configured to administer the amount of apoaequorin to the subject.

Other objects, features and advantages of the present invention will become apparent after review of the specification, claims and drawings.

### BRIEF DESCRIPTION OF THE DRAWINGS

Figure 1 depicts a time-dependent increase in IL-10 mRNA after apoaequorin infusion (A) as a percent of vehicle control. No change in β-actin mRNA (B) was observed. (Apoaequorin is abbreviated as "AQ").
Figure 2 shows (A) the dose-dependent effect of AQ on cell rescue; (B) 4% AQ administered 2 days prior to ischemia is neuroprotective (note the reduction in trypan blue labeled neurons following AQ); (C) the window of neuroprotection provided by AQ; (D) AQ availability after injection into the hippocampus.
Figure 3 depicts (A) IL-10 mRNA expression; (B) β-actin mRNA expression; and (C) PCR arrays displaying changes in multiple cytokines and chemokines following AQ administration.
Figure 4 depicts a graph showing how cytokines are significantly altered with AQ injection.
Figure 5 depicts a schematic showing the interplay of immune response genes examined by the inventors.
Figure 6 depicts graphs showing (A) change in acute inflammatory response genes following AQ injection; (B) change in T/B cell activators following AQ injection; (C) change in angiogenesis/cell proliferator genes following AQ injection; (D) pattern of change in macrophage attracting genes following AQ injection; (E) change in calcium mediator genes following AQ injection; and (F) genes (grouped by function) that change with AQ injection.

### DETAILED DESCRIPTION OF THE INVENTION

### I. IN GENERAL

Before the present materials and methods are described, it is understood that this invention is not limited to the particular methodology, protocols, materials, and reagents described, as these may vary. It is also to be understood that the terminology used herein is for the purpose of describing particular embodiments only, and is not intended to limit the scope of the present invention, which will be limited only by any later-filed nonprovisional applications.

It must be noted that as used herein and in the appended claims, the singular forms "a", "an", and "the" include plural reference unless the context clearly dictates otherwise. As well, the terms "a" (or "an"), "one or more" and "at least one" can be used interchangeably herein. It is also to be noted that the terms "comprising", "including", and "having" can be used interchangeably.

Unless defined otherwise, all technical and scientific terms used herein have the same meanings as commonly understood by one of ordinary skill in the art to which this invention belongs. Although any methods and materials similar or equivalent to those described herein can be used in the practice or testing of the present invention, the preferred methods and materials are now described. All references cited in this specification are to be taken as indicative of the level of skill in the art. Nothing herein is to be construed as an admission that the invention is not entitled to antedate such disclosure by virtue of prior invention.

### II. THE INVENTION

As used herein, "subject" means mammals and non-mammals. "Mammals" means any member of the class Mammalia including, but not limited to, humans, non-human primates such as chimpanzees and other apes and monkey species; farm animals such as cattle, horses, sheep, goats, and swine; domestic animals such as rabbits, dogs, and cats; laboratory animals including rodents, such as rats, mice, and guinea pigs; and the like. Examples of non-mammals include, but are not limited to, birds, and the like. The term "subject" does not denote a particular age or sex.

As used herein, "administering" or "administration" includes any means for introducing apoaequorin into the body, preferably into the brain of the subject, more preferably into the hippocampus of the subject. Examples of administration include but are not limited to oral, nasal, otic, ophthalmic, buccal, sublingual, pulmonary, transdermal, transmucosal, as well as subcutaneous, intraperitoneal, intravenous, epidural and intramuscular injection.

A "therapeutically effective amount" means an amount of apoaequorin that, when administered to a subject for treating a disorder, condition, or disease, is sufficient to effect such treatment for the disorder, or condition, or disease. The "therapeutically effective amount" will vary depending on the disorder, or condition, or disease state being treated, the severity or the disorder, or condition, or disease treated, the age and relative health of the subject, the route and form of administration, the judgment of the attending medical or veterinary practitioner, and other factors.

For purposes of the present invention, "treating" or "treatment" describes the management and care of a patient for the purpose of combating the disease, condition, or disorder. The terms embrace both preventative, i.e., prophylactic, and palliative treatments. Treating includes the administration of apoaequorin to prevent the onset of the symptoms or complications, alleviating the symptoms or complications, or eliminating the disease, condition, or disorder.

The term "apoaequorin" refers to the apoprotein portion of the calcium binding protein aequorin. Aequorin is composed of two distinct units, the respective apoprotein, apoaequorin, with an approximate molecular weight of 22 kDa, and the prosthetic group coelenterazine, a luciferin. Aequorin is a photoprotein isolated from luminescent jellyfish (such as *Aequorea* species, e.g., *Aequorea victoria*) and a variety of other marine organisms. It was originally isolated from the coelenterate by Osamu Shimomura. Apoaequorin useful in the present invention is available from its natural source, via previously known isolation and purification schemes, or publicly known recombinant methods utilizing expression systems which make use of, e.g., recombinant DNA constructs and genetically-modified host cells useful for heterologous expression of apoaequorin.

As used herein, the term "cytokine" shall refer to small cell-signaling protein molecules that are secreted by the glial cells of the nervous system and by numerous cells of the immune system and are a category of signaling molecules used extensively in intercellular communication. Cytokines can be classified as proteins, peptides, or glycoproteins; accordingly, the term "cytokine" encompasses a large and diverse family of regulators produced throughout the body by cells of diverse embryological origin. The term "cytokine" shall encompass immunomodulating agents, such as interleukins and interferons.

As used herein, the term "preconditioning" refers to a technique for producing resistance to the loss of blood supply, and thus oxygen, to tissues of the body. In the present case, preconditioning refers to producing resistance to the loss of blood supply in neurons, as measured by, e.g., assaying percent or number of cells rescued following apoaequorin administration as compared to a control.

The inventors have shown that, when injected directly into dorsal hippocampus (dhpc), the 22 kD CaBP apoaequorin (AQ) derived from the coelenterate *Aequoria victoria* significantly decreases cell death when subjected to an *in vitro* ischemic-type insult (Detert et al., 2009; 2011). This decrease in cell death was present at 24 and 48 hours, but not 1 hour post AQ injection. Interestingly, the AQ protein was present at 1 hour, with significantly less at 24 hours, and by 48 hours post-injection, was barely present. The inverse pattern between AQ's effect and its presence made the inventors question whether or not this change was due to a neuroimmunomodulatory response. Some forms of ischemic preconditioning, such as small ischemic insults, reduce infarcts when given prior to larger, global insults (Ara et al., 2010; Jones & Bergeron, 2001). Ischemic preconditioning's protective effects have been associated with immune system activation (Rehni & Singh, 2012; Wei et al., 2012). While no one mechanism of action is adopted herein, it is possible that if AQ is activating cytokines, cell death decreases by a sort of ischemic preconditioning where the neuroprotective response seen from apoaequorin injections may be due to, in part, a neuroimmunomodulatory response.

Apoaequorin is administered to a patient in a therapeutically effective amount. Apoaequrorin can be administered alone or as part of a pharmaceutically acceptable composition. In addition, apoaequorin or a composition can be administered all at once, as for example, by a bolus injection, multiple times, such as by a series of tablets, or delivered substantially uniformly over a period of time, as for example, using transdermal delivery. Further, the dose of the active agent can be varied over time. Apoaequorin can be administered using an immediate release formulation, a controlled release formulation, or combinations thereof. The term "controlled release" includes sustained release, delayed release, and combinations thereof.

A composition of the invention can be prepared, packaged, or sold in bulk, as a single unit dose, or as a plurality of single unit doses. As used herein, a "unit dose" is discrete amount of the composition comprising a predetermined amount of the active ingredient. The amount of the active ingredient is generally equal to the dosage of the active ingredient that would be administered to a patient or a convenient fraction of such a dosage such as, for example, one-half or one-third of such a dosage.

The relative amounts of the active ingredient, the pharmaceutically acceptable carrier, and any additional ingredients in a composition of the invention will vary, depending upon the identity, size, and condition of the human treated and further depending upon the route by which the composition is to be administered.

Also disclosed herein is a kit comprising a composition of the invention and instructional material. Instructional material includes a publication, a recording, a diagram, or any other medium of expression which is used to communicate the usefulness of the composition of the invention for one of the purposes set forth herein in a human. The instructional material can also, for example, describe an appropriate dose of the pharmaceutical composition of the invention. The instructional material of the kit can, for example, be affixed to a container which contains a pharmaceutical composition of the invention or be shipped together with a container which contains the pharmaceutical composition. Alternatively, the instructional material can be shipped separately from the container with the intention that the instructional material and the pharmaceutical composition be used cooperatively by the recipient.

Also disclosed herein is a kit comprising a composition of the invention and a delivery device for delivering the composition to a human. By way of example, the delivery device can be a syringe, a needle, or a dosage- measuring container. The kit can further comprise an instructional material as described herein. The kit also comprises a container for the separate compositions, such as a divided bottle or a divided foil packet. Additional examples of containers include syringes, boxes, bags, and the like. Typically, a kit comprises directions for the administration of the separate components. The kit form is particularly advantageous when the separate components are preferably administered in different dosage forms (e.g., oral and parenteral), are administered at different dosage intervals, or when titration of the individual components of the combination is desired by the prescribing physician.

Parenteral administration of a pharmaceutical composition includes any route of administration characterized by physical breaching of a tissue of a human and administration of the pharmaceutical composition through the breach in the tissue. Parenteral administration thus includes administration of a pharmaceutical composition by injection of the composition, by application of the composition through a surgical incision, by application of the composition through a tissue-penetrating non-surgical wound, and the like. In particular, parenteral administration includes subcutaneous, intraperitoneal, intravenous, intraarterial, intramuscular, or intrasternal injection and intravenous, intraarterial, or kidney dialytic infusion techniques. For example, the compositions of the present invention can be administered to a subject by brain (via vPAG) injections, intrathecal injections, intraperitoneal injections, or blood injections.

Compositions suitable for parenteral injection comprise the active ingredient combined with a pharmaceutically acceptable carrier such as physiologically acceptable sterile aqueous or nonaqueous solutions, dispersions, suspensions, or emulsions, or may comprise sterile powders for reconstitution into sterile injectable solutions or dispersions. Examples of suitable aqueous and nonaqueous carriers, diluents, solvents, or vehicles include water, isotonic saline, ethanol, polyols (propylene glycol, polyethylene glycol, glycerol, and the like), suitable mixtures thereof, triglycerides, including vegetable oils such as olive oil, or injectable organic esters such as ethyl oleate. Proper fluidity can be maintained, for example, by the use of a coating such as lecithin, by the maintenance of the required particle size in the case of dispersions, and/or by the use of surfactants. Such formulations can be prepared, packaged, or sold in a form suitable for bolus administration or for continuous administration. Injectable formulations can be prepared, packaged, or sold in unit dosage form, such as in ampoules, in multi-dose containers containing a preservative, or in single-use devices for auto-injection or injection by a medical practitioner.

Formulations for parenteral administration include suspensions, solutions, emulsions in oily or aqueous vehicles, pastes, and implantable sustained-release or biodegradable formulations. Such formulations can further comprise one or more additional ingredients including suspending, stabilizing, or dispersing agents. In one embodiment of a formulation for parenteral administration, the active ingredient is provided in dry (i.e. powder or granular) form for reconstitution with a suitable vehicle (e.g. sterile pyrogen-free water) prior to parenteral administration of the reconstituted composition.

The compositions can be prepared, packaged, or sold in the form of a sterile injectable aqueous or oily (emulsion) suspension or solution. This suspension or solution can be formulated according to the known art, and can comprise, in addition to the active ingredient, additional ingredients such as the dispersing agents, wetting agents, or suspending agents described herein. Such sterile injectable formulations can be prepared using a non-toxic parenterally-acceptable diluent or solvent, such as water or 1,3-butanediol, for example. Other acceptable diluents and solvents include Ringer's solution, isotonic sodium chloride solution, and fixed oils such as synthetic mono- or diglycerides. Other parentally-administrable formulations which are useful include those which comprise the active ingredient in microcrystalline form, in a liposomal preparation, or as a component of biodegradable polymer systems. Compositions for sustained release or implantation can comprise pharmaceutically acceptable polymeric or hydrophobic materials such as an emulsion, an ion exchange resin, a sparingly soluble polymer, or a sparingly soluble salt.

Compositions of the invention may also contain adjuvants such as preserving, wetting, emulsifying, and/or dispersing agents, including, for example, parabens, chlorobutanol, phenol, sorbic acid, and the like. It may also be desirable to include isotonic agents, for example, sugars, sodium chloride, and the like. Prolonged absorption of injectable pharmaceutical compositions can be brought about by the use of agents capable of delaying absorption, for example, aluminum monostearate and/or gelatin. In particular, liposomes, mysomes and emulsifiers can be used in to make the present compounds more soluble for delivery.

Dosage forms can include solid or injectable implants or depots. In preferred embodiments, the implant comprises an effective amount of an active agent and a biodegradable polymer. In preferred embodiments, a suitable biodegradable polymer can be selected from the group consisting of a polyaspartate, polyglutamate, poly(L-lactide), a poly(D,L-lactide), a poly(lactide-co-glycolide), a poly(ε-caprolactone), a polyanhydride, a poly(beta-hydroxy-butyrate), a poly(ortho-ester) and a polyphosphazene. In other embodiments, the implant comprises an effective amount of active agent and a silastic polymer. The implant provides the release of an effective amount of

Liquid solutions of the active ingredient in aqueous or oily solvents can be prepared in substantially the same manner as liquid suspensions, the primary difference being that the active ingredient is dissolved, rather than suspended in the solvent. Liquid solutions of the composition of the invention can comprise each of the components described with regard to liquid suspensions, it being understood that suspending agents will not necessarily aid dissolution of the active ingredient in the solvent. Aqueous solvents include, for example, water and isotonic saline. Oily solvents include, for example, almond oil, oily esters, ethyl alcohol, vegetable oils such as arachis, olive, sesame, or coconut oil, fractionated vegetable oils, and mineral oils such as liquid paraffin.

Compositions of the present invention may further include a cyclodextrin component in order to, e.g., improve water solubility of an active pharmaceutical ingredient, prolong drug release, and improve tableting characteristics. In general, cyclic structure oligomers of glucose ("cyclodextrin") are obtained from the starch digests of certain bacteria. The most abundant cyclodextrins are alpha, beta and gamma cyclodextrin which have 6,7 and 8 glucose units, respectively. The interior cavity of a cyclodextrin is hydrophobic and the exposed surface of the molecule is hydrophilic. Cyclodextrins are known to enhance active pharmaceutical ingredient stability, aqueous solubility, and reduce volatility. Some examples of commercially available cyclodextrin, or derivatives thereof, are as follows: alpha-Cyclodextrin (CAS #: 10016-20-3); (2-Hydroxypropyl)-alpha-cyclodextrin (CAS #: 128446-33-3); beta-Cyclodextrin (CAS #: 7585-39-9); 6-O-alpha-D-Glucosyl-beta-cyclodextrin (CAS #: 92517-02-7); gamma-Cyclodextrin (CAS #: 17465-86-0); and (2-Hydroxypropyl)-gamma-cyclodextrin (CAS #: 128446-34-4). Cyclodextrins particularly useful in formulating a delivery vehicle to administer the present active agents include: the sulfobutyl ether beta-cyclodextrin (SBE-beta-CD) available from Cydex Pharmaceuticals, Inc. under the tradename CAPTISOL; and the cyclodextrin and hydroxypropyl betacyclodextrins available from Roquette Pharma under the tradename KLEPTOSE.

For parenteral administration in non-human animals, apoaequorin may be prepared in the form of a paste or a pellet and administered as an implant. Paste formulations can be prepared by dispersing apoaequorin in pharmaceutically acceptable oil such as peanut oil, sesame oil, corn oil or the like. Pellets containing a therapeutically effective amount can be prepared by admixing with a diluent such as a carbowax, carnauba wax, and the like, and a lubricant, such as magnesium or calcium stearate, can be added to improve the pelleting process. It is, of course, recognized that more than one pellet may be administered to an animal to achieve the desired dose level. Moreover, it has been found that such implants may also be administered periodically during the animal treatment period in order to maintain the proper active agent level in the animal's body.

The compositions of the present invention can be administered to a patient at dosage levels in the range of from about 0.01 to about 100 mg/kg per day. For a normal adult human, a dosage in the range of from about 0.01 to about 100 mg/kg is typically sufficient, with 0.1 to 10 mg/kg per day a preferred dosage. However, some variability in the general dosage range may be required depending upon the age and weight of the subject being treated, the intended route of administration, the particular compound being administered and the like. The determination of dosage ranges and optimal dosages for a particular patient is well within the ability of one of ordinary skill in the art having the benefit of the instant disclosure. It is also noted that the compounds of the present invention can be used in sustained release, controlled release, and delayed release formulations, which forms are also well known to one of ordinary skill in the art.

The specific dosage and dosage range that can be used depends on a number of factors, including the requirements of the patient, the severity of the condition being treated, and the pharmacological activity of the active ingredient being administered. The determination of dosage ranges and optimal dosages for a particular patient is well within the ordinary skill of one in the art in view of this disclosure. It is understood that the ordinarily skilled physician, dentist, or veterinarian will readily determine and prescribe an effective amount of the composition to bring about the desired treatment in the subject. In so proceeding, the physician or veterinarian can, for example, prescribe a relatively low dose at first, subsequently increasing the dose until an appropriate response is obtained. It is further understood, however, that the specific dose level for any particular human will depend upon a variety of factors including the activity of the specific composition employed, the age, body weight, general health, gender, and diet of the human, the time of administration, the route of administration, the rate of excretion, any drug combination, and the severity of any disorder being treated.

In certain embodiments, apoaequorin is formulated in the form of a pharmaceutical injectable dosage, including apoaequorin combination with an injectable carrier system. As used herein, injectable and infusion dosage forms (i.e., parenteral dosage forms) include, but are not limited to, liposomal injectables or a lipid bilayer vesicle having phospholipids that encapsulate an active drug substance. Injection includes a sterile preparation intended for parenteral use.

Five distinct classes of injections exist as defined by the USP: emulsions, lipids, powders, solutions and suspensions. Emulsion injection includes an emulsion comprising a sterile, pyrogen-free preparation intended to be administered parenterally. Lipid complex and powder for solution injection are sterile preparations intended for reconstitution to form a solution for parenteral use. Powder for suspension injection is a sterile preparation intended for reconstitution to form a suspension for parenteral use. Powder lyophilized for liposomal suspension injection is a sterile freeze dried preparation intended for reconstitution for parenteral use that is formulated in a manner allowing incorporation of liposomes, such as a lipid bilayer vesicle having phospholipids used to encapsulate an active drug substance within a lipid bilayer or in an aqueous space, whereby the formulation may be formed upon reconstitution. Powder lyophilized for solution injection is a dosage form intended for the solution prepared by lyophilization ("freeze drying"), whereby the process involves removing water from products in a frozen state at extremely low pressures, and whereby subsequent addition of liquid creates a solution that conforms in all respects to the requirements for injections. Powder lyophilized for suspension injection is a liquid preparation intended for parenteral use that contains solids suspended in a suitable fluid medium, and it conforms in all respects to the requirements for Sterile Suspensions, whereby the medicinal agents intended for the suspension are prepared by lyophilization. Solution injection involves a liquid preparation containing one or more drug substances dissolved in a suitable solvent or mixture of mutually miscible solvents that is suitable for injection. Solution concentrate injection involves a sterile preparation for parenteral use that, upon addition of suitable solvents, yields a solution conforming in all respects to the requirements for injections. Suspension injection involves a liquid preparation (suitable for injection) containing solid particles dispersed throughout a liquid phase, whereby the particles are insoluble, and whereby an oil phase is dispersed throughout an aqueous phase or vice-versa. Suspension liposomal injection is a liquid preparation (suitable for injection) having an oil phase dispersed throughout an aqueous phase in such a manner that liposomes (a lipid bilayer vesicle usually containing phospholipids used to encapsulate an active drug substance either within a lipid bilayer or in an aqueous space) are formed. Suspension sonicated injection is a liquid preparation (suitable for injection) containing solid particles dispersed throughout a liquid phase, whereby the particles are insoluble. In addition, the product may be sonicated as a gas is bubbled through the suspension resulting in the formation of microspheres by the solid particles.

The parenteral carrier system includes one or more pharmaceutically suitable excipients, such as solvents and co-solvents, solubilizing agents, wetting agents, suspending agents, thickening agents, emulsifying agents, chelating agents, buffers, pH adjusters, antioxidants, reducing agents, antimicrobial preservatives, bulking agents, protectants, tonicity adjusters, and special additives.

Various exemplary embodiments of compositions according to this invention and methods disclosed herein are now described in the following examples. The following examples are offered for illustrative purposes only and are not intended to limit the scope of the present invention in any way. Indeed, various modifications of the invention in addition to those shown and described herein will become apparent to those skilled in the art from the foregoing description and the following examples and fall within the scope of the appended claims.

### III. EXAMPLES

### Example 1: Time course and effectiveness of apoaequorin in altering expression levels of IL-10.

In this example, the inventors demonstrate the effectiveness of apoaequorin increasing mRNA expression of the anti-inflammatory cytokine IL-10. Preconditioning is a phenomenon in which a brief non-lethal ischemic episode attenuates the damage caused by a subsequent more severe ischemic insult. Preconditioning reduces expression of inflammatory mediators (e.g., IL-1β and IL-6;) compared to that seen in response to brain injury. IL-10 is an anti-inflammatory cytokine and has been shown to be increased following administration of a Ca²⁺-channel blocker. Preconditioning increases the production of IL-10, and IL-10 is associated with neuroprotection. One way it may be neuroprotective is by indirectly reducing IL-6 through reduction of TNF-α production.

### MATERIALS AND METHODS

***Animals.*** 50 male F344 adult rats (mean age = 3.4 ± 0.2 mo.) were used. Rats were kept on a 14/10-hr day/night cycle with free access to food and water.

***Surgery.*** Rats were anesthetized and mounted on a stereotaxic apparatus. Under aseptic conditions, the scalp was incised and retracted to the side, and the head was leveled between bregma and lambda. Each rat was prepared with bilateral stainless steel guide cannulae aimed at the dorsal hippocampus (dhpc) using stereotaxic coordinates (3.5 mm posterior, ± 2.6 mm lateral, 3.0 mm ventral) relative to bregma. Cannulae were secured to the skull with stainless steel screws and epoxy. A stainless steel cap remained in place when the rats were not being injected to prevent the guide cannulae from becoming occluded.

***Drugs.*** Apoaequorin (AQ; 0, 0.4, 1, and 4%, Quincy Bioscience) was prepared in zero Ca²⁺-aCSF (artificial cerebral spinal fluid) with 6% DMSO added to facilitate neuronal uptake. Rats were given an infusion of AQ in one hemisphere and vehicle in the other hemisphere 1 hr, 1 day, 2 days, 3 days, or 5 days prior to decapitation. Bilateral infusions (0.5 µl/side) were given over 60 sec and the injection cannulae remained in place for an additional 2 min to ensure diffusion. The infusion cannulae were cut to extend 0.5 mm beyond the guide cannulae.

***Oxygen-Glucose Deprivation.*** Coronal slices (400 µm) were prepared using standard procedures. Following 1-hour slice recovery in aCSF, *in vitro* ischemia was induced by transferring slices to fructose-CSF (glucose replaced with fructose and bubbled with 95% N₂ - 5% CO₂ instead of a 95% O₂ - 5% CO₂) for 5 min. The slices were then returned to oxygenated aCSF containing 0.2% trypan blue for 30 min reperfusion. Trypan blue readily penetrates dead cells and stains them blue while leaving living cells unstained. The slices were rinsed in oxygenated room temperature aCSF twice then fixed in 10% neutral buffered formalin overnight in the refrigerator. Slices were then cryoprotected, cut on a cryostat (40 µm), and mounted onto subbed slides.

***Cell Counts.*** Slices were examined under an Olympus microscope (equipped with a digital camera) at 10X, and pictures were taken. Trypan blue stained neurons within CA1 (about an 800 µm section) were counted by an experimenter blind to experimental conditions. Statistical analyses were performed using Statview (v 5.0; SAS Institute, Inc., Cary, NC). An ANOVA was used to evaluate a drug effect. Asterisk indicates *p* < .05.

***Western Blots.*** Rats were given a bilateral injection of 4% AQ and brains were removed at one of the following time points: 1 hour, 1 day, 2 days, or 3 days. Brains were rapidly frozen and stored at -80°C. The dhpc and ventral hippocampus (vhpc) were dissected out and homogenized separately. Samples were centrifuged and the supernatant removed and measured using a Bradford protein assay kit (Bio-Rad). Protein samples were normalized and loaded for SDS-PAGE (10%). Proteins were transferred onto PVDF membranes using a semidry transfer apparatus (Bio-Rad). Membranes were then incubated in blocking buffer (2 hours), primary antibody (overnight at 4 °C; 1:5000 mouse anti-aequorin [Chemicon] or 1:1000 rabbit anti-β-actin [Cell Signaling Technology]), and secondary antibody (90 min; 1:5000 goat anti-mouse [Santa Cruz Biotechnology] or 1:5000 goat anti-rabbit [Millipore]). Membranes were then washed, placed in a chemiluminescence solution (Santa Cruz Biotechnology), and exposed to autoradiography film (Hyperfilm MP). Images were taken and densitometry was performed using NIH Image J Software. A band was considered positive if the optical density value of the band (minus the background for each lane) was greater than 2 standard deviations above the mean of the vhpc bands.

***PCR.*** For RT-PCR, rats were given an infusion of 4% AQ in one hemisphere and vehicle in the other hemisphere. One hour, 1 day or 2 days post-infusion, the hippocampi were removed and immediately placed into Trizol. The tissue was homogenized using a 25-gauge needle and syringe, and samples were frozen and stored at -80°C until RNA isolation. RNA was isolated using Qiagen RNeasy mini kit protocol. Isolated RNA was dissolved in 50 µl RNase free H₂O. RNA purity was calculated based on the absorbance ratio of 260 and 280 nm, and an absorbance reading between 1.8 and 2.1 was considered pure RNA. Reverse transcription was conducted to produce cDNA using the Qiagen RT2 HT First Stand Kit. IL-10 and β-actin primers were purchased from Qiagen and used in accordance with the Qiagen RT2 qPCR Primer Assay. Amplification of cDNA was measured by fluorescence of SYBR green (nonspecific DNA stain). Samples were run in triplicate in 96 well plates using StepOne Real Time PCR system and software. Gene expression of the housekeeping protein β-actin, served as a quantification baseline for cytokine expression. Gene expression changes were analyzed using the Pfaffl method. Primer efficiency was calculated based on two randomly selected samples for each of β-actin and IL-10.

### Summary

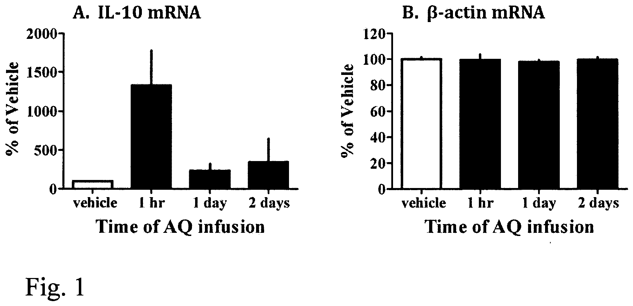

As shown in Figure 1, anti-inflammatory cytokines increased following apoaequorin infusion. In particular, IL-10 mRNA was elevated as early as 1 hour post-infusion. No change in β-actin expression was detected between groups. In view of the well known relationship between increased level of anti-inflammatory cytokines and inflammation reduction, it can be concluded that apoaequorin is a useful agent for reducing inflammation in a medically relevant animal model system, one that is relevant and immediately applicable to the human setting.

### Example 2: Apoaequorin protects neurons from ischemia and alters cytokine mRNA levels in rat hippocampus.

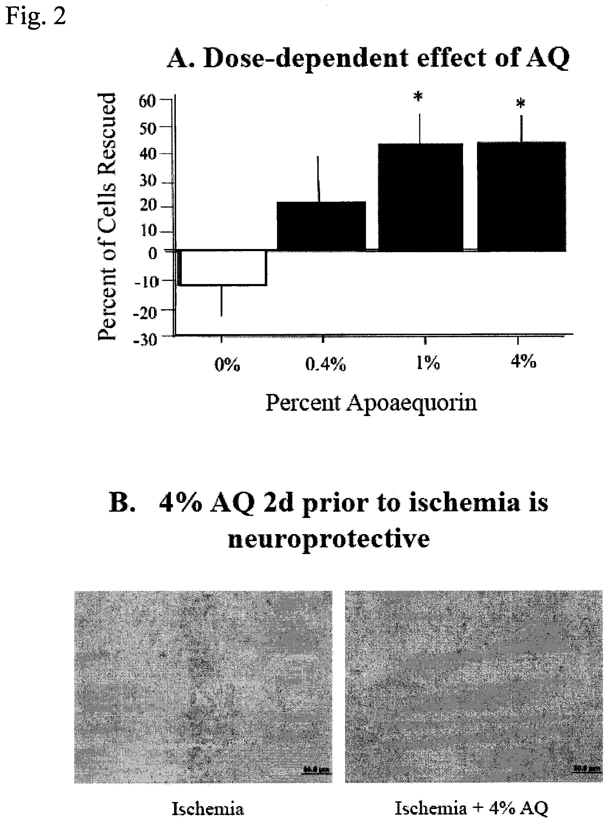

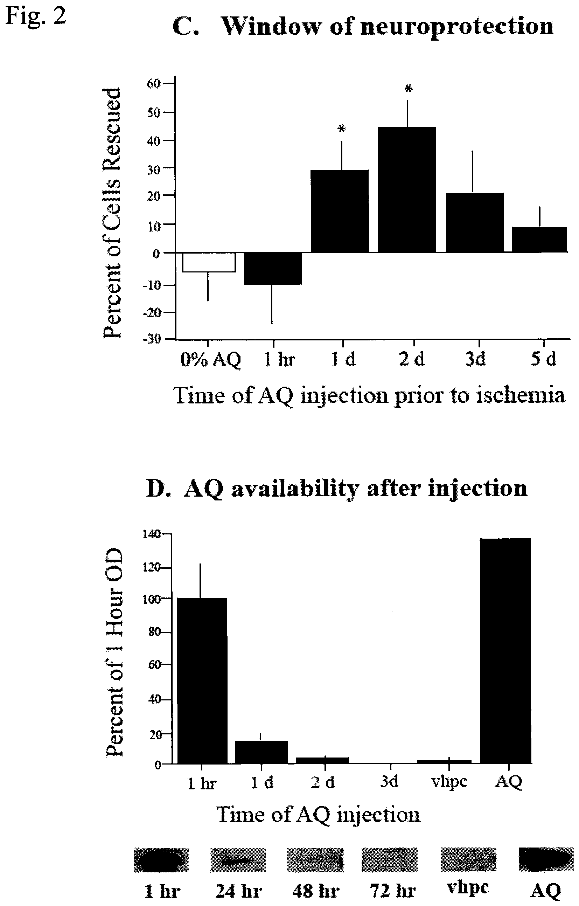

Referring to Fig. 2A, the inventors demonstrated that an intrahippocampal injection of apoaequorin (AQ, the CaBP component of aequorin) significantly protects hippocampal neurons from ischemic cell death. The neuroprotective effects of AQ were first observed at 24 hours and endured for up to 48 hours (Fig. 2B and 2C). Interestingly, western blot analysis suggested that AQ protein levels dramatically decrease over this time period (Fig. 2D). Although the neuroprotective effects of AQ were strongest at 48 hours, the relatively low level of AQ protein present at that time suggests that other factors may play a role in the ability of AQ to protect neurons from ischemic cell death.

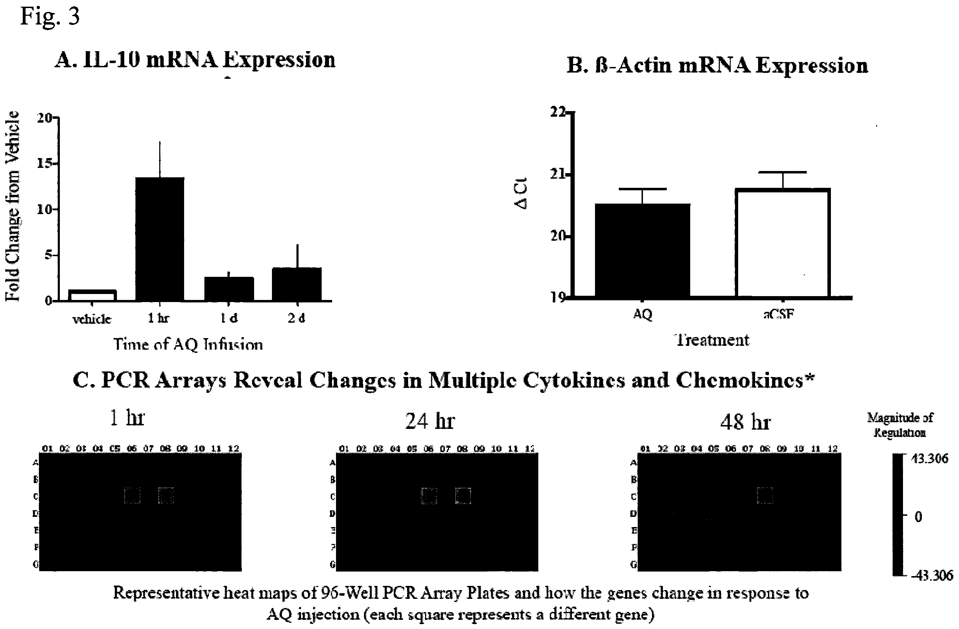

One possibility is that AQ administration leads to a change of levels of anti-inflammatory cytokines. Interleukin-10 (IL-10), an anti-inflammatory cytokine, has previously demonstrated the ability to protect neurons from hypoxia *in vitro.* To test the hypothesis that cytokine expression may play a role in the neuroprotective effects of AQ, cytokine mRNA levels were measured at different time points following a single, intrahippocampal administration of AQ. Twelve male F344 rats were bilaterally cannulated in the dorsal hippocampus. Following recovery, 4% AQ was injected unilaterally (vehicle in the other side, counterbalanced) and after 1, 24, or 48 hrs, the brains were removed and the hippocampi dissected, placed on dry ice, and homogenized. RNA was isolated using Qiagen's RNeasy mini kit. Reverse transcription was performed using Qiagen's RT2 HT First Strand Kit-96 and qPCR was used to quantify IL-10 and β-actin. As shown in Fig. 3A, IL-10 was significantly increased in the AQ treated hemispheres at 1 hour (t(14) = 5.30, *p* < .05), but not 24 or 48 hours (*p* > .05) post injection. AQ treatment did not affect β-actin mRNA levels (Fig. 3B).

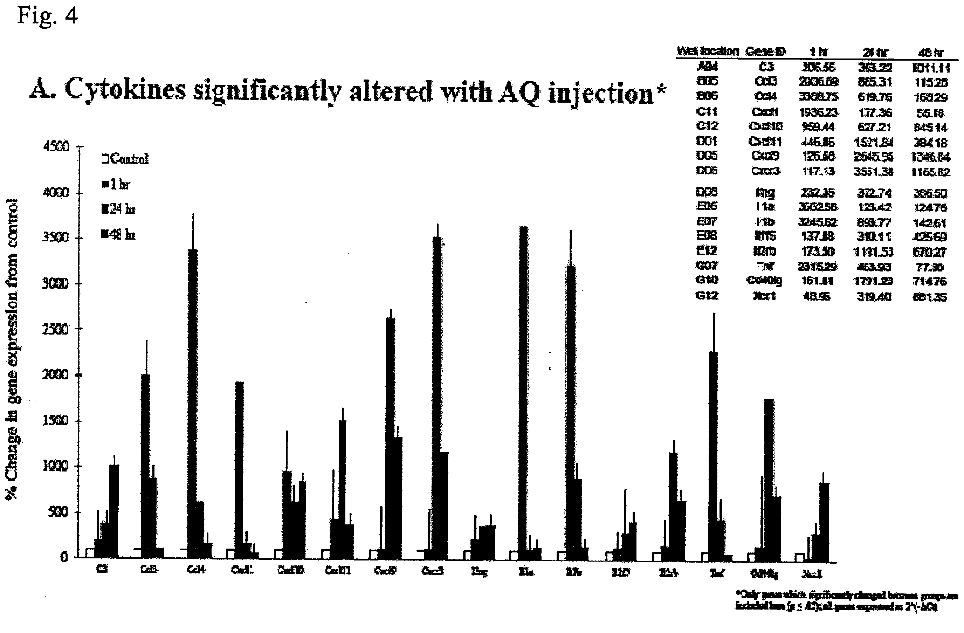

Further PCR array analyses with Qiagen's RT2 Profiler Arrays for cytokines and chemokines were performed in an effort to evaluate whether other cytokines and chemokines are activated at these time points (Fig. 3C). Illustrative results are depicted in Fig. 4 and suggest that multiple anti-inflammatory cytokine and chemokine mRNA levels change in a time-dependent manner following AQ administration.

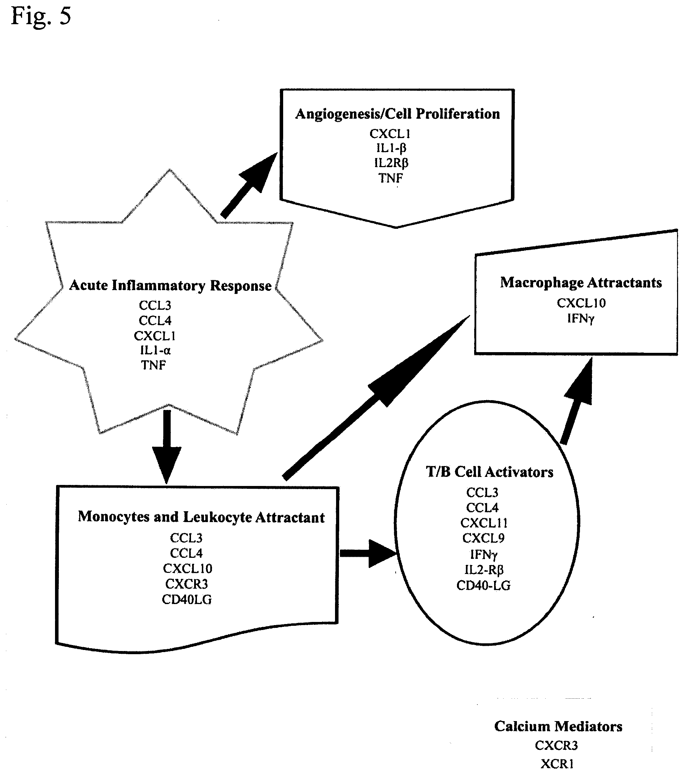

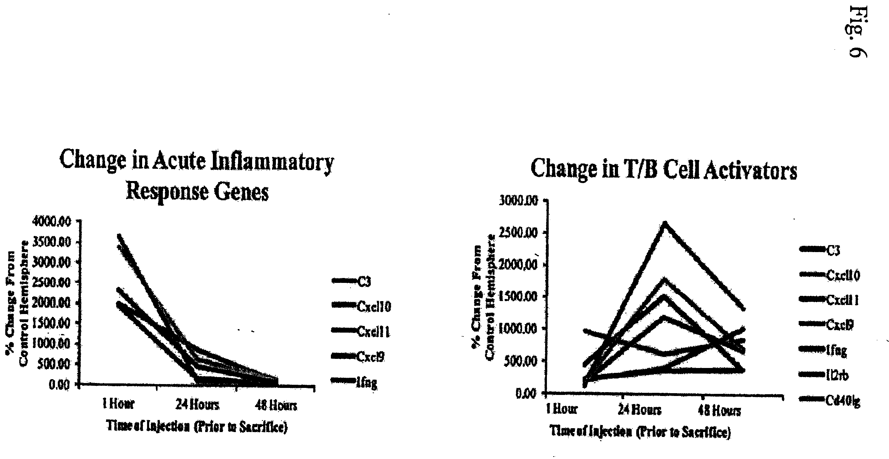

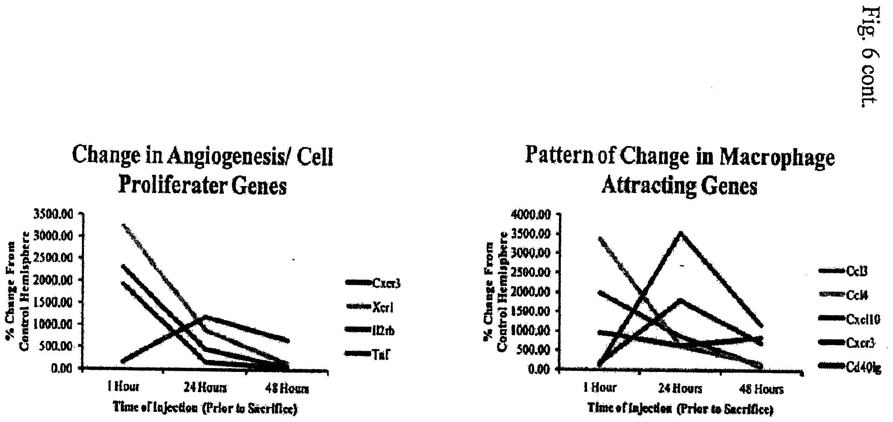

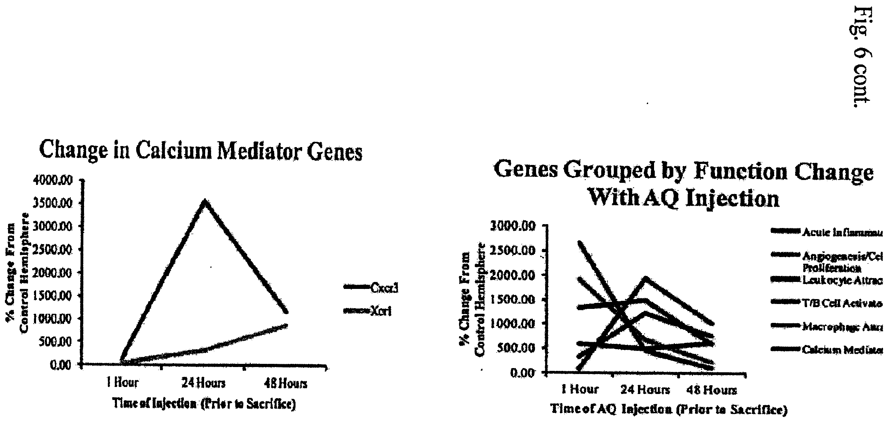

Fig. 5 depicts a schematic showing the interplay of immune response genes examined by the inventors. Accordingly, the inventors parsed the assayed genes by function, and Figure 6 depicts graphs showing (A) change in acute inflammatory response genes following AQ injection; (B) change in T/B cell activators following AQ injection; (C) change in angiogenesis/cell proliferator genes following AQ injection; (D) pattern of change in macrophage attracting genes following AQ injection; (E) change in calcium mediator genes following AQ injection; and (F) genes grouped by function change with AQ injection. Table 1 presents this data in tabular form. Collectively, these results suggest that AQ is neuroprotective and that this neuroprotection involves a neuroimmunomodulatory mechanism.

### Summary

The data presented in this example demonstrate that the neuroprotective effects of apoaequorin are time-dependent. An injection of AQ into area CA1 of dorsal hippocampus significantly decreases cell death when administered 24 and 48, but not 1 hour prior to ischemic insult. Interestingly, AQ shows an inverse relationship between availability and efficacy.

The present data further demonstrate that the neuroprotective effects of apoaequorin may involve a neuroimmunomodulatory mechanism. IL-10 activation post-injection suggests the immune system plays a role in its protective effect. Time-dependent activation of other cytokines and chemokines support this hypothesis.

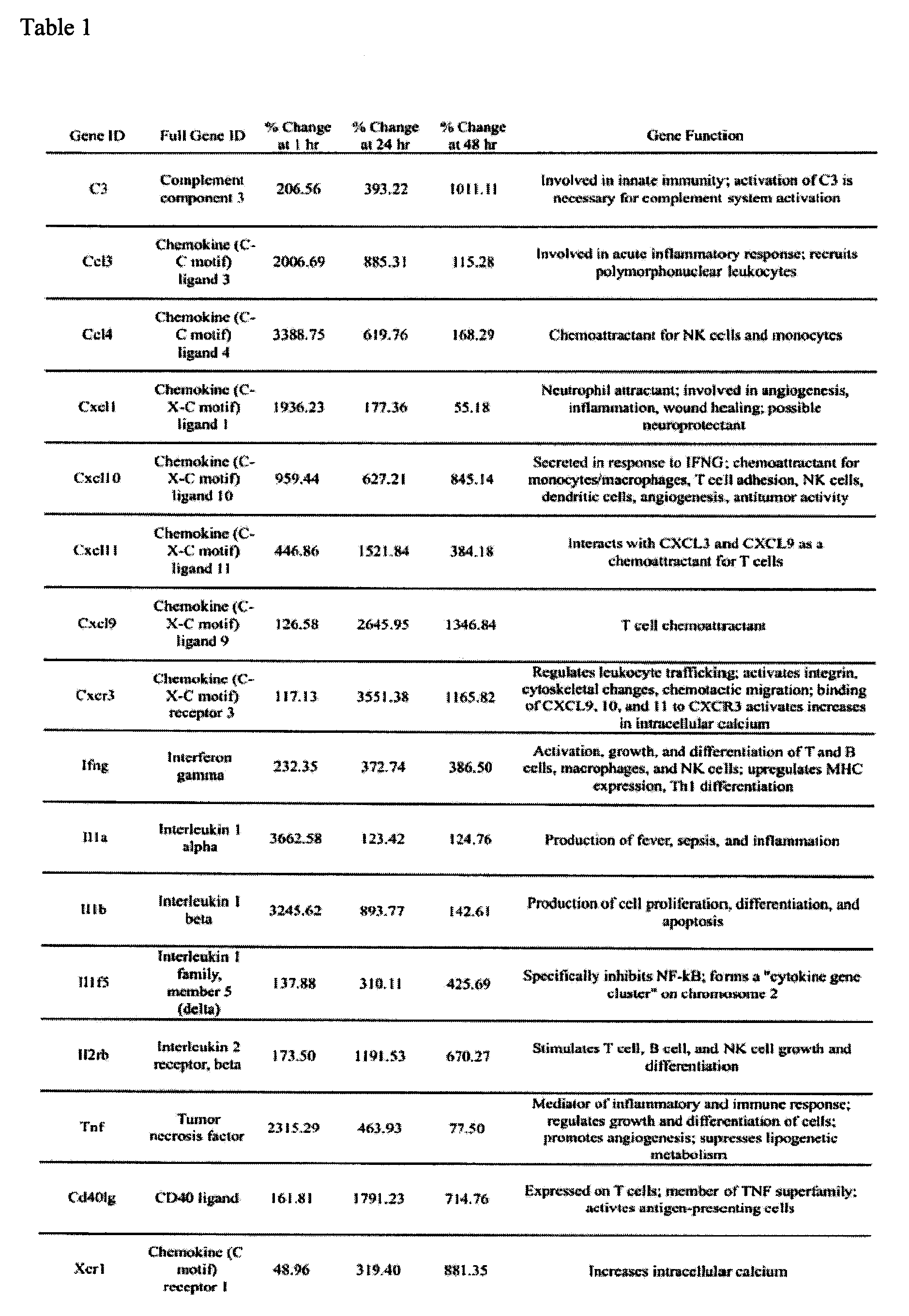

Other embodiments and uses of the invention will be apparent to those skilled in the art from consideration from the specification and practice of the invention disclosed herein. All references cited herein for any reason, including all journal citations and U.S./foreign patents and patent applications, are specifically and entirely incorporated herein by reference. It is understood that the invention is not confined to the specific reagents, formulations, reaction conditions, etc., herein illustrated and described, but embraces such modified forms thereof as come within the scope of the following claims.

### REFERENCES

1. Ara, J., Fekete, S., Frank, M., Golden, J. A., Pleasure, D., Valencia, I. (2011). Hypoxic-preconditioning induces neuroprotection against hypoxia-ischemia in newborn piglet brain. Neurobiology of Disease, 43, 473-485.
2. Baimbridge, K. G., Celio, M. R., & Rogers, J. H. (1992). Calcium-binding proteins in the nervous system. Trends in Neuroscience, 15(8), 303-308
3. Bano, D., Young, K. W., Guerin, C. J., Lefeuvre, R., Rothwell, N. J., Naldini, L., et al. (2005). Cleavage of the plasma membrane Na+/Ca2+ exchanger in excitotoxicity. Cell, 120(2), 275-285.
4. Chard, P. S., Bleakman, D., Christakos, S., Fullmer, C. S., & Miller, R. J. (1993). Calcium buffering properties of calbindin D28k and parvalbumin in rat sensory neurones. Journal ofPhysiology, 472, 341-357.
5. Choi, D. W. (1992). Excitotoxic cell death. Journal of Neurobiology, 23(9), 1261-1276.
6. Detert, J. A., Hochstetter, E. L., Lescher, J. L., Van Langendon, T. M., & Moyer, J. R., Jr. (2011) Time course and effectiveness of aqoaequorin as a neuroprotectant in the brain, Society for Neuroscience Abstracts, Program No. 781.04.
7. Detert, J. A., Heisler, J. D., Hochstetter, E. L., Van Langendon, T. M., & Moyer, J. R., Jr. (2009). Neuroprotection of hippocampal CA1 neurons from ischemic cell death using the calcium binding protein aequorin, Society for Neuroscience Abstracts, 35, Program No. 52.24.
8. Fan, Y., Shi, L., Gu, Y., Zhao, Y., Xie, J., Qiao, J., et al. (2007). Pretreatment with PTD-calbindin D 28k alleviates rat brain injury induced by ischemia and reperfusion. Journal of Cerebral Blood Flow and Metabolism, 27(4), 719-728.
9. Gary, D. S., Sooy, K., Chan, S. L., Christakos, S., & Mattson, M. P. (2000). Concentration- and cell type-specific effects of calbindin D28k on vulnerability of hippocampal neurons to seizure-induced injury. Brain Research. Molecular Brain Research, 75(1), 89-95.
10. Hochstetter, E. L., Detert, J. A., Lescher, J. D., and Moyer, J. R., Jr. (2012). Apoaequorin protects neurons from ischemia and alters cytokine mRNA levels in rat hippocampus. Society for Neuroscience Abstracts, 38, Program No. 860.03.
11. Jones, N. M., & Bergeron, M. (2001). Hypoxic preconditioning induces changes in HIF-1 target genes in neonatal rat brain. Journal of Cerebral Blood Flow and Metabolism, 21(9), 1105-1114.
12. Kristian, T., & Siesjo, B. K. (1998). Calcium in ischemic cell death. Stroke, 29(3), 705-718.
13. Lee, J. M., Zipfel, G. J., & Choi, D. W. (1999). The changing landscape of ischaemic brain injury mechanisms. Nature, 399(6738 Suppl), A7-14.
14. Rehni, A. K., & Singh, T. G. (2012). Involvement of CCR-2 chemokine receptor activation in ischemic preconditioning and postconditioning of brain in mice. Cytokine, 60, 83-89*.*
15. Roger, V. L., Go, A. S., Lloyd-Jones, D. M., Benjamin, E. J., Berry, J. D., Borden, W. B., Bravata, D. M.,...Turner, M. B. (2012). Heart disease and stroke statistics-2012 update: a report from the American Heart Association. Circulation, 125:e2-e220. doi: 10.1161/CIR.0b013e31823ac046.
16. Wei, D., Ren, C., Chen, X., Zhao, H. (2012). The chronic protective effects of limb remote preconditioning and the underlying mechanisms involved in inflammatory factors in rat stroke. PLoS ONE, 7(2): e30892. doi: 10.1371/journal.pone.003892.
17. Yenari, M. A., Minami, M., Sun, G. H., Meier, T. J., Kunis, D. M., McLaughlin, J. R., et al. (2001). Calbindin d28k overexpression protects striatal neurons from transient focal cerebral ischemia. Stroke, 32(4), 1028-1035.

## Claims

1. Apoaequorin for use in preconditioning neurons to reduce neuronal injury due to brain ischemia in a subject by administering the apoaequorin to the subject by injection into area CA1 of dorsal hippocampus 48 hours before the brain ischemia occurs.

2. Apoaequorin for use according to claim 1, wherein the apoaequorin is administered by injection.

3. Apoaequorin for use according to claim 2, wherein the injection is an intra-hippocampal injection.

4. Apoaequorin for use according to claim 1, wherein the subject is human.

5. Apoaequorin for use according to claim 1, wherein the reduction in neuronal injury lasts at least 48 hours from the time of apoaequorin administration.

6. A composition for use in preconditioning neurons in a subject to reduce neuronal injury due to brain ischemia by administering the composition to the subject by injection into area CA1 of dorsal hippocampus 48 hours before the brain ischemia occurs, comprising:
(a) a therapeutically effective dosage of apoaequorin to precondition neurons in a subject to reduce neuronal injury due to brain ischemia; and
(b) a pharmaceutically-acceptable carrier.

7. The composition for use according to claim 6, wherein the composition is formulated as an injectable dosage.

## Patentansprüche

1. Apoaequorin zur Verwendung bei der Präkonditionierung von Neuronen zur Reduzierung neuronaler Schäden aufgrund einer zerebralen Ischämie bei einem Patienten durch Verabreichung des Apoaequorins an den Patienten mittels Injektion in die CA1-Region des dorsalen Hippocampus 48 Stunden vor dem Auftreten der zerebralen Ischämie.

2. Apoaequorin zur Verwendung nach Anspruch 1, wobei die Verabreichung des Apoaequorins mittels Injektion erfolgt.

3. Apoaequorin zur Verwendung nach Anspruch 2, wobei die Injektion eine intrahippocampale Injektion ist.

4. Apoaequorin zur Verwendung nach Anspruch 1, wobei der Patient ein Mensch ist.

5. Apoaequorin zur Verwendung nach Anspruch 1, wobei die Reduzierung neuronaler Schäden ab der Verabreichung des Apoaequorins mindestens 48 Stunden anhält.

6. Zusammensetzung zur Verwendung bei der Präkonditionierung von Neuronen bei einem Patienten zur Reduzierung neuronaler Schäden aufgrund einer zerebralen Ischämie durch Verabreichung der Zusammensetzung an den Patienten mittels Injektion in die CA1-Region des dorsalen Hippocampus 48 Stunden vor dem Auftreten der zerebralen Ischämie, umfassend:
(a) eine therapeutisch wirksame Dosis von Apoaequorin zur Präkonditionierung von Neuronen bei einem Patienten zur Reduzierung neuronaler Schäden aufgrund einer zerebralen Ischämie; und
(b) einen pharmazeutisch verträglichen Trägerstoff.

7. Zusammensetzung zur Verwendung nach Anspruch 6, wobei die Zusammensetzung als injizierbare Darreichungsform formuliert ist.

## Revendications

1. Apoaéquorine pour l'usage dans le préconditionnement de neurones pour réduire des lésions neuronales dues à l'ischémie cérébrale chez un sujet par l'administration de l'apoaéquorine au sujet par injection dans la région CA1 de l'hippocampe dorsal 48 heures avant l'ischémie cérébrale.

2. Apoaéquorine pour l'usage selon la revendication 1, dans laquelle l'apoaéquorine est administrée par injection.

3. Apoaéquorine pour l'usage selon la revendication 2, dans laquelle l'injection est une injection intra-hippocampique.

4. Apoaéquorine pour l'usage selon la revendication 1, dans laquelle le sujet est humain.

5. Apoaéquorine pour l'usage selon la revendication 1, dans laquelle la réduction des lésions neuronales dure au moins 48 heures suivant l'administration de l'apoaéquorine.

6. Composition pour l'usage dans le préconditionnement de neurones chez un sujet pour réduire des lésions neuronales dues à l'ischémie cérébrale par l'administration de la composition au sujet par injection dans la région CA1 de l'hippocampe dorsal 48 heures avant l'ischémie cérébrale, comprenant :
(a) un dosage thérapeutiquement efficace de l'apoaéquorine pour le préconditionnement des neurones chez un sujet pour réduire des lésions neuronales dues à l'ischémie cérébrale ; et
(b) un support pharmaceutiquement acceptable.

7. Composition pour l'usage selon la revendication 6, dans laquelle la composition est formulée comme dosage injectable.
